# EUROPÄISCHE PATENTSCHRIFT

(11) **EP 3 457 972 B1**
(45) Veröffentlichungstag und Bekanntmachung des Hinweises auf die Patenterteilung: **31.07.2024**
(21) Anmeldenummer: 17721154.7
(22) Anmeldetag: 04.05.2017
(51) Int. Cl.: A61B 18/12, A61B 18/14, A61B 90/98, A61B 18/00

(54) **CHIRURGISCHES INSTRUMENT, INSBESONDERE ELEKTROCHIRURGISCHES INSTRUMENT**
SURGICAL INSTRUMENT, IN PARTICULAR ELECTROSURGICAL INSTRUMENT
INSTRUMENT CHIRURGICAL, EN PARTICULIER INSTRUMENT D'ÉLECTROCHIRURGIE

(30) Priorität: 18.05.2016 DE 102016208541
(43) Veröffentlichungstag der Anmeldung: 27.03.2019
(73) Patentinhaber: OLYMPUS Winter & Ibe GmbH, 22045 Hamburg (DE)
(72) Erfinder: GROTH, Carlos, 10439 Berlin (DE); SPRENGER, Christopher, 12163 Berlin (DE)
(74) Vertreter: Seemann & Partner Patentanwälte mbB
(86) Internationale Anmeldenummer: PCT/EP2017/060629
(87) Internationale Veröffentlichungsnummer: WO 2017/198470

(56) Entgegenhaltungen:
- WO-A1-2009/041912
- WO-A2-2015/184446
- US-A- 5 400 267
- US-A1- 2014 305 988
- US-A1- 2015 054 571
- US-A1- 2015 173 947
- US-A1- 2015 216 618
- US-A1- 2015 265 347

## Beschreibung

Die Erfindung betrifft ein chirurgisches Instrument, insbesondere elektrochirurgisches Instrument. Ferner betrifft die Erfindung eine Verwendung einer FRAM-Speichereinrichtung.

Es ist bekannt, dass elektrochirurgische Instrumente beispielsweise bei der elektrochirurgischen Koagulation und/oder der Ablation von biologischem Gewebe zum Einsatz kommen. Dabei werden Elektroden des Instruments mit einer Hochfrequenzspannung (HF-Spannung) unterschiedlichen Potentials (bipolar) beaufschlagt, so dass das die Elektroden umgebene Gewebe soweit erwärmt wird, wodurch körpereigene Eiweiße denaturieren.

Darüber hinaus ist bekannt, dass in der Hochfrequenzchirurgie (auch als HF-Chirurgie bezeichnet) ein hochfrequenter Wechselstrom durch chirurgisch zu behandelndes Gewebe geleitet wird, um dies gezielt zu schädigen oder zu schneiden. Bei dieser Operationstechnik erfolgt gleichzeitig mit dem Schnitt eine Blutstillung durch Verschluss bzw. Verödung der betroffenen Gefäße. Bei der Hochfrequenzchirurgie wird anstatt eines Skalpells eine Elektrode verwendet, die auf ein chirurgisches Instrument, beispielsweise auf ein Resektoskop, aufgesteckt wird.

Ein elektrochirurgisches System umfasst vielfach einen HF-Generator mit einer Leistungsfähigkeit von mehreren 100 Watt und einer Betriebsspannung von 1000 Volt und mehr. Ein von dem HF-Generator erzeugtes HF-Signal wird an die HF-Elektrode angelegt. Bei der monopolaren HF-Chirurgie wird ein Anschluss des Hochspannungsausgangs mit der HF-Elektrode verbunden. Der zweite Anschluss wird mit einer großflächigen Patientenelektrode in Kontakt gebracht. Ausgehend von der HF-Elektrode fließt der zur Behandlung eingesetzte Strom durch den Patienten zurück zu dieser Patientenelektrode. Bei der bipolaren HF-Chirurgie umfasst die zur Behandlung eingesetzte HF-Elektrode zwei Pole, zwischen denen der von dem HF-Generator erzeugte Strom fließt. Während der Hochspannungsentladung bildet sich an der HF-Elektrode ein Plasma, welches die medizinische Behandlung, also Schneidwirkung und/oder Koagulation, bewirkt.

Weiterhin ist bekannt, dass chirurgische Instrumente mit sogenannten RFID-Tags (Radio Frequency Identification) versehen werden, wodurch eine eindeutige Identifizierung des chirurgischen Instruments gewährleistet ist, so dass instrumententypische Informationen in einem RFID-Chip abgespeichert sind. Das Auslesen bzw. Beschreiben des RFID-Chips erfolgt kabelgebunden oder berührungslos.

In WO 2015/184446 A2 ist ein elektrochirurgisches Instrument offenbart, das ein oder mehrere Speichermodule aufweist, wobei hierzu verschiedene Daten über das Instrument im Speichermodul abgespeichert werden. Ferner werden Daten von einem FRAM der bipolaren Vorrichtung gelesen oder darauf geschrieben.

Außerdem ist in US 2014/0305988 A1 ein chirurgisches Instrument mit einem Schaft beschrieben, wobei ein Schaftprozessor mit einer FRAM-Speichereinrichtung vorgesehen ist.

Weiterhin offenbart US 2015/0216618 A1 ein chirurgisches Instrument, wobei das chirurgische Instrument eine Speichereinrichtung umfasst, die einen FRAM-Speicher aufweist.

Darüber hinaus ist in WO 2009/041912 A1 eine Vorrichtung zur Wärmebehandlung von Gewebe beschrieben, wobei eine Laserquelle zur Lieferung von Energien an ein Gewebeerwärmungselement vorgesehen ist. Ferner ist ein Speicher zur Speicherung von über einen Zeitraum hinweg gemessenen Werten vorgesehen.

Des Weiteren offenbart US 2015/0265347 A1 ein elektrochirurgisches Instrument zum Schneiden oder Koagulieren von Gewebe.

Ferner offenbart US 2015/0173947 A1 eine Vorrichtung zur Behandlung eines Augenzustands eines Patienten, wobei eine Versorgungseinheit für eine Sonde einen FRAM-Speicher aufweist.

In US 5,400,267 A ist ein chirurgisches Instrument mit einer Speichervorrichtung zum Erkennen oder Erfassen des chirurgischen Instruments beschrieben.

US 2015/054571 A1 beschreibt ein chirurgisches Instrument mit einem RFID-Tag und einen FRAM-Speicher oder einen EEPROM-Speicher.

Ausgehend von diesem Stand der Technik besteht die Aufgabe der Erfindung darin, die Handhabung von chirurgischen Instrumenten, insbesondere elektrochirurgischen Instrumenten, zu verbessern.

Gelöst wird diese Aufgabe durch ein chirurgisches Instrument, insbesondere elektrochirurgisches Instrument, mit einem RFID-Tag, der mit einer FRAM-Speichereinrichtung zur individuellen Erkennung oder Erfassung des chirurgischen Instruments versehen ist, wobei das chirurgische Instrument ein Kabel, insbesondere ein Stromkabel oder ein Verbindungskabel, aufweist, und das Kabel des chirurgischen Instruments mit einer Verbindungskupplung, insbesondere Stecker, ausgebildet ist, wobei die Verbindungskupplung mit der FRAM-Speichereinrichtung ausgebildet ist.

Dadurch, dass für die Speichereinrichtung eines RFID-Tags des chirurgischen Instruments nicht flüchtige elektronische Speichertypen der FRAM (Ferroelectric Random Access Memory)-Technologie eingesetzt werden, wird ermöglicht, die chirurgischen Instrumente zuverlässig zu identifizieren und beispielsweise deren Behandlungs-und/oder Herstellungshistorie zu dokumentieren. Hierbei ist z.B. der RFID-Tag des chirurgischen Instruments mit einem FRAM-Speicher versehen, wodurch es möglich ist, beispielsweise chirurgische Instrumente, die bislang als Einweg-Instrumente eingesetzt wurden, mittels Gammastrahlung zu sterilisieren, so dass nach der Sterilisierung mittels der Gammastrahlung die vorhandenen im FRAM-Speicher abgespeicherten Informationen nach wie vor vorhanden sind und nicht gelöscht sind und das sterilisierte Instrument wiederverwendet werden kann. Bislang wurden beispielsweise als Einweg-Instrumente bipolare Applikatoren für die Hochfrequenzchirurgie eingesetzt, die nunmehr mit einem FRAM-Speicher ausgebildet sind, wodurch nach der Sterilisierung mittels der Gammastrahlen die vorhandenen Information in dem Speicher und gegebenenfalls das Instrument weiterhin nutzbar sind.

Hierdurch wird eine sichere und automatische, dauerhafte Erkennung des eingesetzten Instruments ermöglicht. Hierbei ist es insbesondere möglich, nach einer Sterilisierung mittels Gammastrahlung das sterilisierte Instrument zu erkennen und für den chirurgischen Einsatz bestimmte chirurgische Instrumente freizuschalten. Darüber hinaus ist es ebenfalls möglich, bei chirurgischen Instrumenten, die als Einweg-Instrumente eingesetzt werden, nach einer vorgenommenen Sterilisierung die Wiederverwendung des mit einem FRAM-Speicher ausgebildeten Instruments zu verhindern, da nach der Gammastrahlensterilisierung das Einweg-Instrument erfasst wird bzw. erkannt wird, und nach einer Erkennung der im FRAM-Speicher gespeicherten Informationen und Daten nicht mehr für eine Wiederverwendung eingesetzt wird.

In einer Ausgestaltung ist vorgesehen, dass die FRAM-Speichereinrichtung in einem Schaft oder in einem Gehäuse des chirurgischen Instruments, z.B. Griff, angeordnet oder eingebaut ist.

Des Weiteren ist es bei dem chirurgischen Instrument vorgesehen, dass das chirurgische Instrument ein Kabel, insbesondere ein Stromkabel oder ein Verbindungskabel, aufweist, wobei die FRAM-Speichereinrichtung im oder am Kabel angeordnet oder vorgesehen ist oder wobei das Kabel mit einer FRAM-Speichereinrichtung ausgebildet ist. Insbesondere ist hierbei das Kabel dauerhaft mit dem chirurgischen Instrument verbunden, wodurch eine eindeutige Identifikation des chirurgischen Instruments erreicht wird.

Das Kabel des chirurgischen Instruments ist mit einer Verbindungskupplung, insbesondere Stecker, ausgebildet, wobei die Verbindungskupplung mit der FRAM-Speichereinrichtung ausgebildet ist. Hierbei ist die FRAM-Speichereinrichtung in oder an der Verbindungskupplung angeordnet oder vorgesehen. Insbesondere weist ein als Verbindungskupplung ausgebildeter Stecker eine FRAM-Speichereinrichtung auf, die vorzugsweise im Inneren des Steckers bzw. im Gehäuse des Steckers angeordnet ist.

Des Weiteren ist vorzugsweise das chirurgische Instrument als, vorzugsweise bipolarer, Applikator zur elektrochirurgischen Koagulation und/oder zur Ablation von biologischem Gewebe ausgebildet. Hierbei werden die als Handinstrumente ausgebildeten bipolaren Applikatoren in der Hochfrequenz (HF)-Chirurgie oder Radiofrequenz (RF)-Chirurgie eingesetzt. Ferner ist es im Rahmen der Erfindung möglich, dass ein monopolarer Hochfrequenz (HF)-Applikator oder Ultraschall-Applikator oder Mikrowellen-Applikator eine FRAM-Speichereinrichtung aufweist.

Ferner wird die Aufgabe gelöst durch die Verwendung einer FRAM-Speichereinrichtung bei einem chirurgischen Instrument oder in einem chirurgischen Instrument, insbesondere elektrochirurgischen Instrument, das voranstehend beschrieben worden ist. Zur Vermeidung von Wiederholungen wird auf die obigen Ausführungen ausdrücklich verwiesen.

Weitere Merkmale der Erfindung werden aus der Beschreibung erfindungsgemäßer Ausführungsformen zusammen mit den Ansprüchen und der beigefügten Zeichnung ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand eines Ausführungsbeispiels unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigt:
- Fig. 1: schematisch ein elektrochirurgisches Instrument und
- Fig. 2a, 2b: schematisch einen Stecker eines elektrochirurgi-sches Instruments in einer Draufsicht (Fig. 2a) und in einem Querschnitt (Fig. 2b).

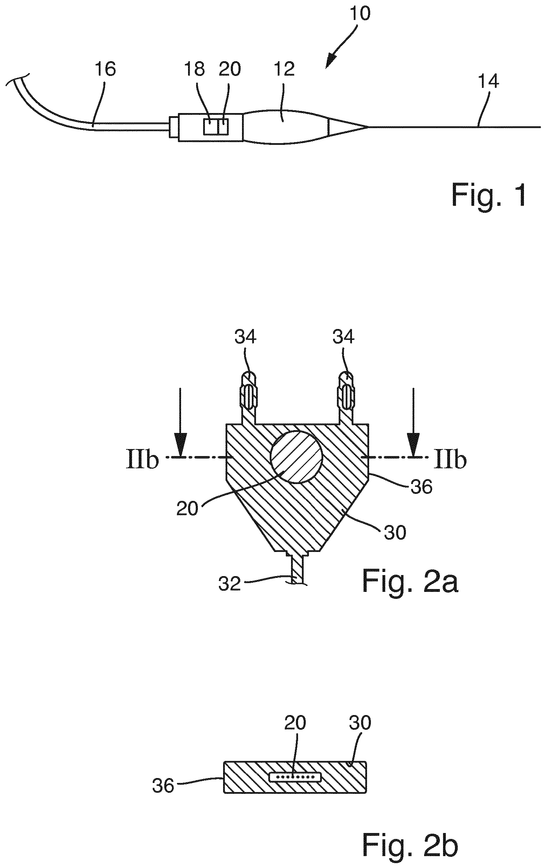

In Fig. 1 ist schematisch ein elektrochirurgisches Instrument 10 gezeigt. Das elektrochirurgische Instrument 10 ist dabei vorzugsweise als bipolares elektrochirurgisches Instrument ausgebildet.

Das elektrochirurgische Instrument 10 umfasst einen schaftförmigen Griff 12, an dessen einem Ende eine Applikatornadel 14 angeordnet ist. Die Applikatornadel 14 ist an der Spitze mit zwei Koagulationselektroden ausgebildet, die durch einen Isolator voneinander elektrisch getrennt sind. Mittels der Koagulationselektroden ist es möglich, biologisches Gewebe zu schneiden und zu koagulieren.

Zum Betrieb des elektrochirurgischen Instruments 10 ist am von der Applikatornadel 14 abgewandten Ende des Griffes 12 das chirurgische Instrument 10 mittels einer Leitung 16 mit einer Versorgungseinheit verbunden. Mittels der Leitung 16 werden bei angeschlossenem chirurgischen Instrument 10 die Koagulationselektroden mit einer Spannung beaufschlagt.

Darüber hinaus kann der Griff 12 für den Betrieb des chirurgischen Instruments 10 entsprechende Schalter bzw. Aktivierungsschalter aufweisen.

Ferner ist vorgesehen, dass im Griff 12 ein RFID-Tag 18 angeordnet ist, der mit einem schematisch eingezeichneten FRAM-Speicher (Ferroelectric Random Access Memory) 20 ausgebildet ist. Mittels des FRAM-Speichers 20 ist es möglich, individuelle Eigenschaften des chirurgischen Instruments 10 zu speichern und diesen bei Bedarf mittels einer Ausleseeinheit auszulesen. Hierdurch wird eine sichere automatische Erkennung des chirurgischen Instruments 10 ermöglicht. Insbesondere ist es durch die Verwendung des FRAM-Speichers 20 möglich, das chirurgische Instrument 10 mittels Gammastrahlung zu sterilisieren, ohne dass während bzw. nach der Sterilisierung Daten aus dem FRAM-Speicher 20 gelöscht werden oder gelöscht sind.

In einer Alternative ist es möglich, dass das RFID-Tag 18 mit dem FRAM-Speicher 20 an der Leitung 16 angeordnet ist, um eine Identifikation des chirurgischen Instruments 10 zu erreichen.

In Fig. 2a ist schematisch eine Draufsicht auf einen Anschlussstecker 30 eines (hier nicht dargestellten) elektrochirurgischen Instruments gezeigt. Fig. 2b zeigt schematisch einen Querschnitt durch den Stecker 30 gemäß der in Fig. 2a gestrichelten Linie IIb - IIb.

Mittels des Anschlusssteckers 30 wird beispielsweise ein Hochfrequenzgerät (für die Elektrochirurgie) mit einem chirurgischen Instrument verbunden. Hierzu ist der Anschlussstecker 30 mit einem Anschlusskabel 32, z.B. einem HF-Anschlusskabel, verbunden, um Strom, insbesondere HF-Strom von einem Hochfrequenzgerät zu dem elektrochirurgischen Instrument zu leiten. Der als Verbindungskupplung ausgebildete Anschlussstecker 30 weist an seiner Einsteckseite als Kontakte oder Kontaktstifte zwei Einsteckstifte 34 auf, die z.B. in eine Steckaufnahme eines Hochfrequenzgeräts oder dergleichen eingesteckt werden oder einsteckbar sind. Von weiteren Einzelheiten wurde aus Gründen der besseren Darstellung abgesehen.

Der Anschlusssteckers 30 weist ein, vorzugsweise isolierendes, Gehäuse 36 auf, in dem ein FRAM-Speicher 20 angeordnet oder aufgenommen ist. Im Inneren des Gehäuses 36 ist der FRAM-Speicher 20 von einem Ummantelungsmaterial des Steckers 30 umgeben. Mittels der im FRAM-Speicher 20 gespeicherten Daten und Informationen ist es möglich, das mit dem Anschlussstecker 30 versehene und verbundene chirurgische Instrument zuverlässig zu identifizieren und beispielsweise dessen Behandlungs- und/oder Herstellungshistorie zu dokumentieren.

Der in den Fig. 2a und 2b gezeigte Anschlussstecker 30 mit dem FRAM-Speicher 20 ist als ein Flachstecker ausgebildet. Im Rahmen der Erfindung kann der Anschlussstecker 30 auch nur einen Steckstift 34 oder mehr als zwei Einsteckstifte, d.h. wenigstens einen oder mehrere Einsteckstifte, aufweisen. Ferner kann der Anschlussstecker 30 mit einer anderen Form als die Flachstecker-Form ausgebildet sein.

Im Rahmen der Erfindung sind Merkmale, die mit "insbesondere" oder "vorzugsweise" gekennzeichnet sind, als fakultative Merkmale zu verstehen.

### Bezugszeichenliste

- 10: elektrochirurgisches Instrument
- 12: Griff
- 14: Applikatornadel
- 16: Leitung
- 18: RFID-Tag
- 20: FRAM-Speicher
- 30: Anschlussstecker
- 32: Anschlusskabel
- 34: Einsteckstift
- 36: Gehäuse

## Patentansprüche

1. Chirurgisches Instrument (10), insbesondere elektrochirurgisches Instrument, mit einem RFID-Tag (18), der mit einer FRAM-Speichereinrichtung (20) zur individuellen Erkennung oder Erfassung des chirurgischen Instruments (10) versehen ist, wobei das chirurgisches Instrument (10) ein Kabel, insbesondere ein Stromkabel oder ein Verbindungskabel, aufweist, und das Kabel des chirurgischen Instruments (20) mit einer Verbindungskupplung, insbesondere Stecker, ausgebildet ist, wobei die Verbindungskupplung mit der FRAM-Speichereinrichtung (20) ausgebildet ist.

2. Chirurgisches Instrument (10) nach Anspruch 1, **dadurch gekennzeichnet, dass** die Verbindungskupplung als Anschlussstecker (30) ausgebildet ist.

3. Chirurgisches Instrument (10) nach Anspruch 2, **dadurch gekennzeichnet, dass** der Anschlussstecker (30) ein isolierendes Gehäuse (36) aufweist.

4. Chirurgisches Instrument (10) nach Anspruch 3, **dadurch gekennzeichnet, dass** in dem Gehäuse (36) die FRAM-Speichereinrichtung (20) aufgenommen ist.

5. Chirurgisches Instrument (10) nach einem der Ansprüche 2 bis 4, **dadurch gekennzeichnet, dass** der Anschlussstecker (30) als Flachstecker ausgebildet ist.

6. Chirurgisches Instrument (10) nach einem der Ansprüche 2 bis 5, **dadurch gekennzeichnet, dass** der Anschlussstecker (30) einen Einsteckstift (34) oder zwei Einsteckstifte (34) oder mehr als zwei Einsteckstifte (34) aufweist.

7. Chirurgisches Instrument (10) nach einem der Ansprüche 1 bis 6, **dadurch gekennzeichnet, dass** das chirurgische Instrument (10) als, vorzugsweise bipolarer, Applikator zur elektrochirurgischen Koagulation und/oder zur Ablation von biologischem Gewebe ausgebildet ist.

8. Verwendung einer FRAM-Speichereinrichtung (20) bei einem chirurgischen Instrument (10), insbesondere elektrochirurgischen Instrument, nach einem der Ansprüche 1 bis 7 zur individuellen Erkennung oder Erfassung des chirurgischen Instruments.

## Claims

1. A surgical instrument (10), in particular electrosurgical instrument, having an RFID tag (18) which is provided with an FRAM storage device (20) for individually recognizing or detecting the surgical instrument (10), wherein the surgical instrument (10) has a cable, in particular a power cable or a connecting cable, and the cable of the surgical instrument (20) being designed with a connecting coupling, in particular a plug, wherein the connecting coupling is designed with the FRAM storage device (20).

2. The surgical instrument (10) according to claim 1, **characterized in that** the connecting coupling is designed as a connector plug (30).

3. The surgical instrument (10) according to claim 2, **characterized in that** the connecting plug (30) has an insulating housing (36).

4. The surgical instrument (10) according to claim 3, **characterized in that** the FRAM storage device (20) is accommodated in the housing (36).

5. The surgical instrument (10) according to any one of claims 2 to 4, **characterized in that** the connector plug (30) is designed as a flat plug.

6. The surgical instrument (10) according to any one of claims 2 to 5, **characterized in that** the connector plug (30) has a one plug-in pin (34) or two plug-in pins (34) or more than two plug-in pins (34).

7. The surgical instrument (10) according to any one of claims 1 to 6, **characterized in that** the surgical instrument (10) is configured as a, preferably bipolar, applicator for electrosurgically coagulating and/or for ablating biological tissue.

8. A use of a FRAM storage device (20) with a surgical instrument (10), in particular an electrosurgical instrument, according to any one of claims 1 to 7 for individually recognizing or detecting the surgical instrument (10).

## Revendications

1. Instrument chirurgical (10), en particulier instrument électrochirurgical, avec une étiquette RFID (18) qui est pourvue d'un dispositif de mémoire FRAM (20) pour la reconnaissance ou l'enregistrement individuel de l'instrument chirurgical (10), l'instrument chirurgical (10) présentant un câble, en particulier un câble électrique ou un câble de connexion, et le câble de l'instrument chirurgical (20) étant réalisé avec un raccord de connexion, en particulier un connecteur, le raccord de connexion étant formé avec le dispositif de mémoire FRAM (20).

2. Instrument chirurgical (10) selon la revendication 1, **caractérisé en ce que** le raccord de connexion est réalisé sous la forme d'une fiche de raccordement (30).

3. Instrument chirurgical (10) selon la revendication 2, **caractérisé en ce que** la fiche de connexion (30) présente un boîtier isolant (36).

4. Instrument chirurgical (10) selon la revendication 3, **caractérisé en ce que** le dispositif de mémoire FRAM (20) est logé dans le boîtier (36).

5. Instrument chirurgical (10) selon l'une des revendications 2 à 4, **caractérisé en ce que** la fiche de raccordement (30) est conçue sous la forme d'une fiche plate.

6. Instrument chirurgical (10) selon l'une des revendications 2 à 5, **caractérisé en ce que** la fiche de raccordement (30) présente une broche d'insertion (34) ou deux broches d'insertion (34) ou plus de deux broches d'insertion (34).

7. Instrument chirurgical (10) selon l'une des revendications 1 à 6, **caractérisé en ce que** l'instrument chirurgical (10) est conçu sous la forme d'un applicateur, de préférence bipolaire, pour la coagulation électrochirurgicale et/ou l'ablation de tissus biologiques.

8. Utilisation d'un dispositif de mémorisation FRAM (20) sur un instrument chirurgical (10), notamment électrochirurgical, selon l'une des revendications 1 à 7, pour la reconnaissance ou la détection individuelle de l'instrument chirurgical.
